(19) Europäisches Patentamt
European Patent Office
Office européen des brevets

(11) Publication number: 0 183 802
B1

(12) # EUROPEAN PATENT SPECIFICATION

(45) Date of publication of patent specification: 05.09.90

(51) Int. Cl.[5]: A 61 K 33/42, A 61 K 33/10, A 61 K 33/08

(21) Application number: 85902917.5

(22) Date of filing: 03.06.85

(86) International application number: PCT/US85/01038

(87) International publication number: WO 85/05552 19.12.85 Gazette 85/27

(54) # HIGH-DENSITY ANTACID POWDERS.

(30) Priority: 04.06.84 US 617008

(43) Date of publication of application: 11.06.86 Bulletin 86/24

(45) Publication of the grant of the patent: 05.09.90 Bulletin 90/36

(84) Designated Contracting States: AT BE CH DE FR GB IT LI LU NL SE

(56) References cited:
US-A-2 880 136
US-A-2 923 660
US-A-3 245 876
US-A-3 272 703
US-A-3 573 006
US-A-3 591 680
US-A-3 879 525
US-A-4 145 400

No relevant documents have been disclosed

(73) Proprietor: REHEIS, INC.
235 Snyder Avenue
Berkeley Heights New Jersey 07922 (US)

(72) Inventor: ABBRUSCATO, Victor, J.
8618 15th Avenue
Brooklyn, NY 11228 (US)
Inventor: NELSON, Roger, E.
1526 Leon Drive
Hatfield, PA 19440 (US)

(74) Representative: Lehn, Werner, Dipl.-Ing. et al
Hoffmann, Eitle & Partner Patentanwälte
Arabellastrasse 4
D-8000 München 81 (DE)

EP 0 183 802 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to improved antacid compositions. More particularly, this invention relates to high-density antacid powders capable of being compounded into standard dosage forms, such as tablets and capsules, having increased amounts of active antacid per dose and a process for preparing same.

Antacid compositions are designed for relieving hyperacidity and associated conditions caused by excess acid in the stomach. Such an antacid composition, inter alia, should neutralize an adequate amount of gastric hydrochloric acid and maintain its action during the period of gastric digestion; adequate and repeated doses should be palatable to the hyperacid patient; it should not have side effects such as gastric irritation; and it should maintain its antacid properties upon ageing so that the proper amount of active agent is ingested on extended use.

Antacid compositions have been prepared in a variety of forms such as solutions, suspensions, emulsions, powders, capsules and tablets. The composition in each case includes an active antacid substance such as aluminum hydroxide-magnesium carbonate co-dried gel, dihydroxyaluminum sodium carbonate, magnesium trisilicate, aluminum hydroxide, aluminum basic carbonates and magnesium hydroxide. The beneficial response of conditions and diseases treated with antacid compositions is in direct proportion to the amount of antacid ingested and its duration of action with the stomach.

The tablet or capsule form of antacid composition is more convenient than antacid liquid compositions to the patient. These may be carried in a small package and may be taken as needed without any need for extra equipment, such as a spoon, for the measuring of dosages. The convenience of these solid forms of the antacid makes them a popular kind of antacid composition. However, the solid antacid products of the prior art contain relatively small amounts of active antacid ingredients per tablet or capsule. And since the size of the capsules or tablets may not be increased substantially without rendering them too large for human consumption, it is necessary to repeat doses and increase frequency or ingestion to achieve the desirable therapeutic results. However, a higher density antacid powder would allow a smaller capsule or tablet size.

Another advantage for the use of high-density antacid powders is that they are more flowable and directly compressible than lower density powders. Because of this property, the powders can be more easily processed into capsules and tablets, and they can be processed using a direct compression method. This is a less expensive method than the wet granulation method, typically used to process lower density powders.

US—A—2 880 136 discloses a co-precipitate of aluminum hydroxide with magnesium carbonate and/or calcium carbonate, which is obtained by co-precipitating salts of aluminum and one or both of the metals magnesium and calcium with soda.

US—A—3 272 703 discloses an antacid composition comprising a magnesium and/or calcium aluminum hydroxy carbonate co-precipitate wherein the co-precipitation is carried out at a pH of about 9 to about 10.

It is the object of the present invention to provide solid antacic compositions having high bulk or apparent densities, said densities being measured before compression.

It is another object of the present invention to provide a method for the preparation of antacid powders having said high bulk or apparent densities.

The invention comprises an aluminum magnesium antacid powder having a bulk or apparent density $\geqq 0.5$ prior to compression gram/cm$^2$ comprising on a percent by weight basis:

25—40% MgO;
15—25% $AL_2O_3$; and
6—20% A;

wherein A represents an anionic species or a mixture of anionic species.

The high-density antacid powders of the present invention are produced by a process comprising the steps of:

a) preparing an aqueous solution of a water-soluble aluminum compound and at least one water-soluble magnesium compound which can partially be replaced by a water-soluble calcium compound;

b) precipitating the magnesium (calcium) aluminum compounds simultaneously with a solution of a base selected from alkali metal hydroxides and ammonium hydroxide at a pH of not exceeding 8.5; and

c) making the precipitate into a powder having a density $\geqq 0.5$ gram/cm$^2$.

Various combinations of metal hydroxides are known in the prior art and are used for various purposes, among others, as therapeutic agents for treating stomach hyperacidity. These and other types of antacid compounds can be produced in the form of powders which in turn can be formed into tablets and capsules of proper dosage. The antacid powders so produced normally have an apparent density in the range of about 0.1 to 0.4 g/cm$^3$.

Applicants have discovered a process by which antacid powders having an apparent density $\geqq 0.5$ g/cm$^3$ may be produced. The use of such high density powders advantageously provides for the delivery of higher levels of active antacids in standard size tablets or capsules.

In the practice of the present invention any water-soluble aluminum compounds may be used, such as an aluminum halide, alkali metal aluminate, aluminum nitrate and aluminum sulfate. Similarly, any water-soluble magnesium compound can be used, such as a magnesium halide, magnesium nitrate and magnesium sulfate.

In another embodiment of the present invention, it is contemplated to include water-soluble calcium compounds to produce an antacid powder wherein all or part of the magnesium is replaced by calcium.

The anionic species represented by A include $OH^-$, $Cl^-$, $HCO_3^-$, $CO_3^=$, $HSO_4^-$, $SO_4^=$, $HPO_4^{2-}$, $H_2PO_4^-$ and $PO_4^{\equiv}$.

In carrying out the process of the present invention, aqueous solutions of the reactants are mixed to precipitate the desired antacid complex, and the precipitate is formed into a powder as follows:

Aqueous solutions of an aluminum compound, a magnesium compound an sodium hydroxide are simultaneously metered into a reactor at a rate to maintain the pH below 8.5 to precipitate the magnesium-aluminum antacid. For convenience, the aluminum and magnesium compound solutions can be mixed before metering into the reactor. The maintenance of the pH below 8.5 is critical in the precipitation process in order to obtain the high-density antacid powder. Normally, the reactants are added at a rate to maintain the pH between 7.5 and 8.5. This pH range was found optimal for obtaining antacid powders having a density in the range of 0.5 to 1.1 g/cm$^3$. Above the pH of 8.5 powder densities decrease, while below 7.5 the washing time of the precipitate becomes excessive.

A post-precipitation step to prevent the loss of soluble magnesium may be included in the process. This post-precipitation step is carried out with the addition of a base, normally a sodium, potassium, or ammonium hydroxide solution, to raise the pH to 9—10, without any detriment to the powder density. A solution of a salt such as $Na_2CO_3$, $NaHCO_3$, $Na_2SO_4$, $NaHSO_4$, $NaH_2PO_4$, $Na_2HPO_4$ and $Na_3PO_4$ can be added with mixing to the magnesium-aluminum precipitate to substitute the anion carried by the precipitate with an anion from the added salt. For example, if the starting magnesium or aluminum compounds are chlorides, bromides or fluorides, the chloride, bromide or fluoride ions will be substituted with anions such as carbonate, sulfate or phosphate.

The precipitate obtained, with or without the inclusion of the post-precipitation steps, is washed and formed into a cake having a thick gel-like consistency. The cake is then repulped and dried at a temperature not exceeding about 150°C. Drying is accomplished using methods and equipment used in the art of processing materials into a powder, such as oven drying, freeze drying or spray drying.

Another important aspect to the process of the present invention is the use of an alkali metal hydroxide or ammonium hydroxide as the base in the constant-pH precipitation process, instead of sodium carbonate or sodium bicarbonate, although small amounts of carbonates or bicarbonates can be tolerated. It has been found that precipitation with an alkali metal hydroxide or ammonium hydroxide produces the desired high-density antacid powders, while precipitation with alkali carbonates or bicarbonates results in the production of antacid powders having densities less than 0.5 g/cm$^3$. This and other aspects of the pesent invention will be further illustrated by the examples that follow.

## Example 1

3000 grams of an aqueous solution of $AlCl_3$, $\rho = 1.20$ (24° Baume) were mixed with 3630 grams of a 50% $MgCl_2 \cdot 6H_2O$ solution. 4.5 liters of water were added to a 20-liter reactor, and an overhead mixer was set up and turned on to ensure proper mixing. The mixed chloride solution and 6.3 liters of a 14.7% NaOH solution were simultaneously metered into the 20-liter reactor. The rates were adjusted to hold a constant pH of 8.0 with a total precipitation time of 46 minutes. The precipitation slurry was divided into parts A, B, C and D. Part A had no post-precipitation addition; parts B and C had additions of NaOH solution to raise the pH to 9.0 and 10.0, respectively. Part D had an addition of 0.5 liter of a $Na_2CO_3$-solution, $p = 1.12$ (16° Baume) with an end pH of 9.5. The slurries in each part were then washed, repulped and spray dried at an outlet temperature of 125°C to a powder. The results obtained are shown in Table I.

TABLE I

| Properties | Formulas A | B | C | D |
|---|---|---|---|---|
| % w/w $Al_2O_3$ | 23.4 | 22.6 | 21.7 | 20.9 |
| % w/w MgO | 30.9 | 32.6 | 32.5 | 31.6 |
| % w/w Na | 0.13 | 0.13 | 0.24 | 0.26 |
| % w/w Cl | 8.58 | 8.85 | 9.30 | 1.44 |
| % w/w $CO_2$ | 1.47 | 1.93 | 1.33 | 6.57 |
| Apparent Density, g/cm³ | 1.0 | 1.0 | 1.0 | 0.68 |
| pH Stat* at 37°C., $T_{50}$-min. | 2.8 | 2.8 | 2.3 | 3.2 |
| $T_{90}$-min. | 10.3 | 12.3 | 11.3 | 5.3 |
| ACC**, ml 0.1N HCl/g | 251 | 261 | 257 | 275 |
| ACCD***, ml 0.1N HCl/cm³ | 251 | 261 | 257 | 187 |

The pH stat* test here and hereinbelow is a measure of antacid rate of reactivity and total neutralization capacity.

ACC** means acid-consuming capacity as determined by the pH-stat titration.

ACCD*** is the ACC multiplied by the apparent density.

The pH-stat titration is performed as follows. A weight of sample containing exactly 500 mg total oxide ($Al_2O_3$ + MgO) is suspended in 300 ml of deionized water. The suspension is maintained at 37°C±1° and stirred at 300 rpm. The sample is titrated with 1.0N HCl at such a rate as to maintain a constant pH of 3.0, while recording on a strip chart the volume of acid used versus time. The titration is continued until no more acid is consumed. The times, in minutes, when 50% ($T_{50}$) and 90% ($T_{90}$) of the total antacid are consumed by the acid are determined. The ACC is calculated by dividing the total number of millimeters (times 10) of 1.0N HCl by the weight of the sample in grams.

The apparent density is determined by transferring 15—20 grams of sample to a 250-ml, flat bottomed, graduated cylinder. The cylinder is given 60 taps (1 sec. lift, 1 sec. drop; 2.54 cm drop), and the volume in cubic centimeters is read. The apparent density (g/cm³) is equal to the weight of the sample (g) divided by the measured volume of the sample (cm³).

In the following examples, the same procedures are followed to determine apparent density and pH-stat reactivity and total neutralization capacity.

Example 2 (outside the invention)

The precipitation process of Example 1 was repeated except that instead of sodium hydroxide a sodium carbonate solution was used as the base in the precipitation, and the precipitation slurry so obtained was divided into parts E and F. Part E of the precipitation slurry had no post-addition treatment, while part F had a post addition of NaOH solution to raise the pH to 9.0. The results are shown in Table II.

TABLE II

| Properties | Formula E | Formula F |
|---|---|---|
| % w/w $Al_2O_3$ | 40.7 | 23.8 |
| % w/w MgO | 10.12 | 24.5 |
| % w/w Na | 0.23 | 0.21 |
| % w/w Cl | 0.11 | 0.11 |
| Apparent Density, g/cm$^3$ | 0.42 | 0.24 |
| pH Stat* at 37°C., $T_{50}$-min. | 0.75 | 0.35 |
| $T_{90}$-min. | 2.33 | 1.66 |
| ACC**, ml 0.1N HCl/g | 309 | 281 |
| ACCD***, ml 0.1N HCl/cm$^3$ | 130 | 67 |

The results show that the powders produced, using $Na_2CO_3$ for precipitation, have densities less than 0.5 g/cm$^3$, and also, powder E has a low concentration of MgO which is the result of soluble magnesium loss.

Example 3

3.7 Liters of a $MgSO_4$ solution, ρ = 1.31 (34° Baume) was mixed with 2.5 liters of a 24° Baume $AlCl_3$ solution. 4.5 Liters of water was placed in a 20-liter reactor which was fitted with an overhead mechanical mixer. The acid mixture and 5.1 liters of a 14.7% NaOH solution were simultaneously metered into the reactor. The flow rate was adjusted to hold a constant pH of 7.4 and a precipitation time of 48 minutes. The slurry obtained was divided into parts G, H, I and J. Part G had no post addition; parts H and I had additions of respectively 1.0 and 1.3 liters of a $Na_2CO_3$-solution, ρ = 1.12 (16° Baume); and part J had a post addition of NaOH solution to raise the pH to 9.0. The results are shown in Table III.

TABLE III

| Properties | Formulas G | H | I | J |
|---|---|---|---|---|
| % w/w $Al_2O_3$ | 24.9 | 21.5 | 21.0 | 18.5 |
| % w/w MgO | 26.1 | 30.1 | 30.6 | 31.8 |
| % w/w Na | 0.36 | 0.1 | 0.08 | 0.29 |
| % w/w Cl | 0.51 | 0.19 | 0.21 | 0.42 |
| % w/w $SO_4$ | 14.41 | 9.43 | 8.22 | 14.34 |
| % w/w $CO_2$ | 0.85 | 4.7 | 5.9 | 1.59 |
| Apparent Density, g/cm$^3$ | 0.74 | 0.59 | 0.54 | 0.83 |
| pH Stat* at 37°C., $T_{50}$-min. | 1.4 | 0.3 | 0.33 | 1.7 |
| $T_{90}$-min. | 4.33 | 1.0 | 0.85 | 4.4 |
| ACC**, ml 0.1N HCl/g | 247 | 259 | 259 | 239 |
| ACCD***, ml 0.1N HCl/cm$^3$ | 183 | 153 | 140 | 198 |

The addition of $Na_2CO_3$ increased the amount of % $CO_2$ in Formulas H and I while decreasing the amount of sulfate.

Example 4

The precipitation process of Example 1 is repeated except that the rates were adjusted to hold a constant pH of 8.9. The precipitation slurry was divided into parts K, L, M and N. Part K had no post-precipitation addition; parts L, M and N had additions of 0.25, 0.35 and 0.50 liter respectively of a $Na_2CO_3$-solution, ρ = 1.12 (16° Baume). The final pH's were 9.5, 10.2 and 10.5, respectively. The results obtained are shown in Table IV.

TABLE IV

| Properties | Formulas K | L | M | N |
|---|---|---|---|---|
| % w/w $Al_2O_3$ | 21.2 | 20.4 | 20.9 | 20.0 |
| % w/w MgO | 32.2 | 32.1 | 32.1 | 32.3 |
| % w/w Na | 0.02 | 0.03 | 0.09 | 0.05 |
| % w/w Cl | 9.21 | 2.73 | 0.95 | 0.22 |
| % w/w $CO_2$ | 2.60 | 6.50 | 8.33 | 8.64 |
| Apparent Density, g/cm³ | 0.38 | 0.28 | 0.30 | 0.30 |
| pH Stat* at 37°C., $T_{50}$-min. | 3.6 | 1.3 | 1.6 | 1.3 |
| $T_{90}$-min. | 17 | 12.3 | 12.0 | 10.7 |
| ACC**, ml 0.1N HCl/g | 263 | 282 | 278 | 282 |
| ACCD***, ml 0.1N HCl/cm³ | 100 | 79 | 83 | 85 |

The results show that the powders produced at a precipitation pH above 8.5 have apparent densities less than 0.5 g/cm³ and correspondingly low ACCD values.

Example 5

The precipitation process of Example 1 was repeated. The precipitation slurry was divided into parts O, P, Q and R. Part O had no post-precipitation addition; parts P and Q had additions respectively of 300 ml and 600 ml of a 14.5% $Na_2SO_4$ solution; part R had an addition of 0.5 liter of a $Na_2CO_3$-solution, ρ = 1.12 (16° Baume). The results are shown in Table V.

TABLE V

| Properties | Formulas O | P | Q | R |
|---|---|---|---|---|
| % w/w $Al_2O_3$ | 22.3 | 21.2 | 20.3 | 20.3 |
| % w/w MgO | 32.5 | 35.1 | 32.1 | 32.8 |
| % w/w Na | 0.07 | 0.13 | 0.19 | 0.19 |
| % w/w Cl | 8.77 | 3.55 | 1.39 | 0.85 |
| % w/w $SO_4$ | 0.27 | 9.10 | 13.65 | 0.47 |
| % w/w $CO_2$ | 1.88 | 1.62 | 1.19 | 8.80 |
| Apparent Density, g/cm$^3$ | 0.97 | 0.94 | 0.89 | 0.59 |
| pH Stat* at 37°C., $T_{50}$-min. | 1.55 | 1.0 | 0.7 | 1.0 |
| $T_{90}$-min. | 3.66 | 3.7 | 2.3 | 4.3 |
| ACC**, ml 0.1N HCl/g | 245 | 246 | 245 | 275 |
| ACCD***, ml 0.1N HCl/cm$^3$ | 238 | 231 | 218 | 162 |

Table V demonstrates that the process according to the present invention can be used to produce high density aluminum magnesium hydroxide sulfate or carbonate powders by exchanging these ions of chloride. Starting with the initial chloride precipitation (O), sulfate (P, Q), or carbonate (R) is exchanged for chloride by the post-precipitation addition of sodium sulfate or sodium carbonate.

Example 6

3000 grams of an aqueous solution of $AlCl_3$, ρ = 1.20 (24° Baume), 2928 grams of a 50% $MgCl_2 \cdot 6H_2O$ solution and 533 grams of a 50% $CaCl_2 \cdot 2H_2O$ solution were added to a 20 liter reactor and reacted with 14.7% NaOH solution as in Example 1. The precipitation slurry so obtained was divided into parts S, T, U, and V. Part S had no post-precipitation addition, parts T, U, and V had additions of 0.25, 0.35, and 0.50 liter respectively of a $Na_2CO_3$-solution, ρ = 1.12 (16° Baume). The powder results are shown in Table VI.

TABLE VI

| Properties | Formulas S | T | U | V |
|---|---|---|---|---|
| % w/w $Al_2O_3$ | 25.7 | 24.2 | 22.7 | 20.8 |
| % w/w MgO | 29.3 | 29.1 | 27.4 | 25.5 |
| % w/w CaO | 0.5 | 1.88 | 4.88 | 7.79 |
| % w/w Na | 0.044 | 0.041 | 0.083 | 0.097 |
| % w/w Cl | 7.43 | 1.49 | 1.33 | 1.03 |
| Apparent Density, g/cm$^3$ | 1.09 | 0.88 | 0.71 | 0.68 |
| pH Stat* at 37°C., $T_{50}$-min. | 4.2 | 2.5 | 1.8 | 1.8 |
| $T_{90}$-min. | 13 | 6.5 | 6.0 | 3.3 |
| ACC**, ml 0.1N HCl/g | 249 | 285 | 284 | 276 |
| ACCD***, ml 0.1N HCl/cm$^3$ | 271.4 | 250.8 | 201.6 | 187.7 |

This example shows the incorporation of calcium into high-density aluminum magnesium antacid powders.

The high-density powders of the present invention prepared as above described may be compressed into tablets or filled into capsules of desired form using standard tableting and capsuling procedures, including wet granulation and direct compression.

**Claims**

1. An aluminum magnesium antacid powder having a bulk or apparent density ≧ 0.5 prior to compression gram/cm³ comprising on a percent by weight basis:

25—40% $MgO$;
15—25% $AL_2O_3$; and
6—20% A;

wherein A represents an anionic species or a mixture of anionic species.

2. An aluminum magnesium antacid powder of claim 1 having a bulk or apparent density prior to compression in the range of 0.5 to 1.1 gram/cm³.

3. The aluminum magnesium antacid powder of Claim 1, wherein said anionic species is selected from $OH^-$, $Cl^-$, $HCO_3^-$, $CO_3^=$, $HSO_4^-$, $SO_4^=$, $H_2PO_4^-$, $H_2PO_4^=$ and $PO_4^{\equiv}$.

4. The antacid powder of Claim 1 wherein part of the magnesium oxide is replaced by calcium oxide.

5. An antacid complex powder according to claim 1 wherein the powder prior to compression has an acid-consuming capacity per unit density of at least 140 ml 0.1 N HCl/cm³.

6. A method for the preparation of an antacid powder having an apparent density ≧ 0.5 gram/cm³ comprising the steps of:

a) preparing an aqueous solution of a water-soluble aluminum compound and at least one water-soluble magnesium compound which can partially be replaced by a water-soluble calcium compound;

b) precipitating the magnesium (calcium) aluminum compounds simultaneously with a solution of a base selected from alkali metal hydroxides and ammonium hydroxide at a pH of not exceeding 8.5; and

c) making the precipitate into a powder having a density ≧ 0.5 gram/cm³.

7. The method of Claim 6 further comprising a post-precipitation step of adding a basic solution to the precipitate to raise the pH to 9—10 thereof.

8. The method of Claim 6 further comprising a post-precipitation step of adding an alkali or ammonium salt selected from $HCO_3^-$, $CO_3^=$, $HSO_4^-$, $SO_4^=$, $H_2PO_4^-$, $HPO_4^{2-}$, and $PO_4^{3-}$ to introduce the anion of the salt into the antacid precipitate.

9. The method of Claim 7 or 8 wherein said precipitate is washed, repulped, and dried into a powder.

10. The method of Claim 5 wherein said magnesium compound is selected from magnesium halide, magnesium sulfate, magnesium nitrate and mixtures thereof.

11. The method of Claim 6 wherein said aluminum compound is selected from aluminum halides, alkali metal aluminates, aluminum nitrate, aluminum sulfate and mixtures thereof.

12. The method of Claim 6 wherein said pH is between about 7.5—8.5.

13. The method of Claim 6 wherein said base is sodium hydroxide, potassium hydroxide or ammonium hydroxide.

**Patentansprüche**

1. Ein Aluminium-Magnesium-Antacid-Pulver mit einer Schüttdichte oder scheinbaren Dichte ≧ 0,5 vor dem Zusammenpressen g/cm³, umfassend auf einer Gewichtsprozentbasis:

25 bis 40% $MgO$;
15 bis 25% $Al_2O_3$; und
6 bis 20% A;

worin A eine Anionspezies oder eine Mischung von Anionspezies bedeutet.

2. Ein Aluminium-Magnesium-Antacid-Pulver gemäß Anspruch 1 mit einer Schüttdichte oder scheinbaren Dichte vor der Kompression im Bereich von 0,5 bis 1,1 g/cm³.

3. Das Aluminium-Magnesium-Antacid-Pulver gemäß Anspruch 1, bei dem die Anionenspezies aus $OH^-$, $Cl^-$, $HCO_3^-$, $CO_3^=$, $HSO_4^-$, $SO_4^=$, $H_2PO_4^-$, $HPO_4^=$, und $PO_4^=$ ausgewählt sind.

4. Das Antacid-Pulver gemäß Anspruch 1, bei dem ein Teil des Magnesiumoxids durch Calciumoxid ersetzt ist.

5. Ein Antacid-Komplex-Pulver gemäß Anspruch 1, bei dem das Pulver vor der Kompression eine Säure verbrauchende Kapazität pro Einheitsdichte von wenigstens 140 ml 0,1 N HCl/cm³ hat.

6. Verfahren zur Herstellung eines Antacid-Pulvers mit einer scheinbaren Dichte von ≧ 0,5 g/cm³, umfassend die Stufen:

(a) Herstellen einer wäßrigen Lösung aus einer wasserlöslichen Aluminiumverbindung und wenigstens einer wasserlöslichen Magnesiumverbindung, die teilweise durch eine wasserlösliche Calciumverbindung ersetzt werden kann;

(b) Ausfällen der Magnesium-(Calcium)-Aluminium-Verbindungen gleichzeitig mit einer Lösung aus einer Base ausgewählt aus Alkalihydroxiden und Ammoniumhydroxid bei einem pH-Wert, der 8,5 nicht übersteigt, und

(c) Verarbeiten des Niederschlags zu einem Pulver einer Dichte $\geqq$ 0,5 g/cm$^3$.

7. Verfahren gemäß Anspruch 6, welches weiterhin eine Nachausfällstufe umfaßt, bei der man eine basische Lösung zu dem Niederschlag gibt, um dessen pH-Wert auf 9 bis 10 zu erhöhen.

8. Verfahren gemäß Anspruch 6, umfassend weiterhin eine Nachausfällstufe, bei der man eine Alkali- oder Ammoniumsalz, ausgewählt aus $HCO_3^-$, $CO_3^=$, $HSO_4^-$, $SO_4^=$, $H_2PO_4^-$, $HPO_4^{2-}$ und $PO_4^{3-}$ zugibt, um das Anion des Salzes in das Antacid-Präzipitat einzuführen.

9. Verfahren gemäß Anspruch 7 oder 8, bei dem das Präzipitat gewaschen, wieder aufgeschwämmt und zu einem Pulver getrocknet wird.

10. Verfahren gemäß Anspruch 5, bei dem die Magnesiumverbindung ausgewählt ist aus Magnesiumhalogenid, Magnesiumsulfat, Magnesiumnitrat und Mischungen davon.

11. Verfahren gemäß Anspruch 6, bei dem Aluminiumverbindung ausgewählt ist aus Aluminiumhalogeniden, Alkalialuminaten, Aluminiumnitrat, Aluminiumsulfat und Mischungen davon.

12. Verfahren gemäß Anspruch 6, bei dem der pH-Wert zwischen 7,5 bis 8,5 beträgt.

13. Verfahren gemäß Anspruch 6, bei dem die Base Natriumhydroxid, Kaliumhydroxid oder Ammoniumhydroxid ist.

**Revendications**

1. Pourdre d'antiacide à base d'aluminium et de magnésium ayant une densité apparente supérieure ou égale à 0,5 gramme/cm$^3$ avant la compression, comprenant en pourcentage pondéral:

25—40% de MgO;
15—25% d'$Al_2O_3$; et
6—20% de A;

où A représente une espèce anionique ou un mélange d'espèces anioniques.

2. Pourdre d'antiacide à base d'aluminium et de magnésium selon la revendication 1, ayant une densité apparente avant la compression dans l'intervalle de 0,5 à 1,1 gramme/cm$^3$.

3. Pourdre d'antiacide à base d'aluminium et de magnésium selon la revendication 1, dans laquelle cette espèce anionique est choisie parmi $OH^-$, $Cl^-$, $HCO_3^-$, $CO_3^{2-}$, $HSO_4^-$, $SO_4^{2-}$, $HPO_4^{2-}$, $H_2PO_4^-$ et $PO_4^{3-}$.

4. Poudre d'antiacide selon la revendication 1, dans laquelle une partie de l'oxyde de magnésium est remplacée par de l'oxyde de calcium.

5. Poudre complexe d'antiacide selon la revendication 1, dans laquelle la poudre, avant compression, a une capacité de consommation d'acide par unité de densité d'au moins 140 ml de HCl 0,1 N/cm$^3$.

6. Procédé pour la préparation d'une poudre d'antiacide ayant une densité apparente $\geq$ 0,5 gramme/cm$^3$, comprenant les étapes de:

a) préparation d'un solution aqueuse d'un composé de l'aluminium soluble dans l'eau et d'au moins un composé du magnésium soluble dans l'eau qui peut être remplacé partiellement par un composé du calcium soluble dans l'eau;

b) précipitation simultanée des composés du magnésium (calcium) et de l'aluminium avec une solution d'une base choisie parmi les hydroxydes de métaux alcalins et l'hydroxyde d'ammonium à un pH ne dépassant pas 8,5; et

c) transformation du précipité en une poudre ayant une densité supérieure ou égale à 0,5 gram/cm$^3$.

7. Procédé selon la revendication 6, comprenant en outre une étape, après précipitation, d'addition d'une solution basique au précipité pour élever le pH à 9—10.

8. Procédé selon la revendication 6, comprenant en outre une étape, après précipitation, d'addition d'une sel alcalin ou d'ammonium d'un anion choisi parmi $HCO_3^-$, $CO_3^{2-}$, $HSO_4^-$, $SO_4^{2-}$, $H_2PO_4^-$, $HPO_4^{2-}$, et $PO_4^{3-}$ pour introduire l'anion du sel dans le précipité d'antiacide.

9. Procédé selon les revendication 7 ou 8, dans lequel ledit précipité est lavé, redispersé et séché sous forme de poudre.

10. Procédé selon la revendication 5, dans lequel ledit composé du magnésium est choisi parmi un halogénure de magnésium, le sulfate de magnésium, le nitrate de magnésium et des mélanges de ceux-ci.

11. Procédé selon la revendication 6, dans lequel ledit composé de l'aluminium est choisi parmi des halogénures, d'aluminium, des aluminates de métaux alcalins, le nitrate d'aluminium, le sulfate d'aluminium et des mélanges de ceux-ci.

12. Procédé selon la revendication 6, dans lequel ledit pH est compris entre environ 7,5 et 8,5.

13. Procédé selon la revendication 6, dans lequel ladite base est l'hydroxyde de sodium, l'hydroxyde de potassium ou l'hydroxyde d'ammonium.